# EUROPÄISCHE PATENTSCHRIFT

(11) **EP 3 298 345 B1**
(45) Veröffentlichungstag und Bekanntmachung des Hinweises auf die Patenterteilung: **20.03.2019**
(21) Anmeldenummer: 16729501.3
(22) Anmeldetag: 20.05.2016
(51) Int. Cl.: A61B 1/00, A61B 1/04, A61B 1/06, A61C 9/00, A61B 1/247, G01B 11/24, G01B 11/25

(54) **KAMERA UND VERFAHREN ZUR DREIDIMENSIONALEN VERMESSUNG UND FARBVERMESSUNG EINES DENTALEN OBJEKTS**
CAMERA AND METHOD FOR THE THREE-DIMENSIONAL MEASUREMENT AND COLOUR MEASUREMENT OF A DENTAL OBJECT
CAMÉRA ET PROCÉDÉ POUR RÉALISER UNE MESURE EN 3D ET UNE MESURE DE COULEUR D'UN OBJET DENTAIRE

(30) Priorität: 22.05.2015 DE 102015209410
(43) Veröffentlichungstag der Anmeldung: 28.03.2018
(73) Patentinhaber: Sirona Dental Systems GmbH, 64625 Bensheim (DE)
(72) Erfinder: THIEL, Frank, 64372 Ober-Ramstadt (DE); PFEIFFER, Joachim, 64625 Bensheim (DE)
(74) Vertreter: Sommer, Peter
(86) Internationale Anmeldenummer: PCT/EP2016/061368
(87) Internationale Veröffentlichungsnummer: WO 2016/188879

(56) Entgegenhaltungen:
- WO-A1-2010/145669
- WO-A1-2012/083967
- WO-A1-2014/125037
- WO-A1-2014/202442

## Beschreibung

### Technisches Gebiet

Die Erfindung betrifft eine Kamera zur dreidimensionalen Vermessung eines dentalen Objekts, aufweisend mit mindestens einer Lichtquelle, die einen Beleuchtungsstrahl abstrahlt, mindestens eine Projektionsmaske, die ein Projektionsmuster erzeugt, eine Fokussierungsoptik, die das Projektionsmuster in einer scharfen Ebene in einem festgelegten Fokusabstand relativ zur dentalen Kamera abbildet, wobei das auf das Objekt projizierte Projektionsmuster als ein Beobachtungsstrahl vom Objekt zurückgestrahlt wird und mittels eines Sensors aufgenommen wird.

### Stand der Technik

Aus dem Stand der Technik sind mehrere Verfahren und Kameras zur dreidimensionalen Vermessung von dentalen Objekten bekannt.

WO 2014/125037 A1 offenbart eine Vermessungsvorrichtung zur Vermessung von Oberflächen und einer Oberflächenfarbe eines Objekts, wobei eine mehrfarbige Lichtquelle, ein Farbsensor und ein Datenverarbeitungssystem verwendet werden, um aus mehreren 2D-Bildern ein dreidimensionales Modell des Objekts zu berechnen.

WO 2014/202442 A1 offenbart eine Vermessungsvorrichtung zur farbkodierten Triangulation, wobei ein Muster auf das Objekt projiziert wird und mittels eines Sensors in mehreren Spektralbereichen vermessen wird. Vor dem Sensor sind dabei mehrere Farbfilter für die einzelnen Spektralbereich angeordnet.

In der WO 2012/083967 A1 ist eine Vorrichtung zur optischen 3D-Vermessung eines Objekts unter Verwendung einer optischen konfokalen Messmethode offenbart, wobei zusätzlich zu einer ersten Lichtquelle mindestens eine zweite Lichtquelle verwendet wird, deren Licht unter Verwendung eines Lichtleiters in den Strahlengang der Vorrichtung eingekoppelt wird. Darüber hinaus ist offenbart, dass die Lichtquellen, wie farbliche LED's oder LED's in Kombination mit Farbfiltern, verwendet werden können, wobei die Lichtquellen abwechselnd eingeschaltet werden, um eine homogene Ausleuchtung zu gewährleisten.

Die WO 2010/145669 A1 offenbart eine Vorrichtung zur optischen 3D-Vermessung eines Objekts unter Verwendung einer optischen konfokalen Messmethode. Dabei wird ein sich zeitlich änderndes Muster auf das Objekt projiziert. Das sich ändernde Muster wird mittels eines motorbetriebenen mechanischen Mittels in Form eines Rads erzeugt.

Ein Nachteil dieser Verfahren besteht darin, dass das sich zeitlich ändernde Projektionsmuster unter Verwendung beweglicher Projektionsmittel im Beleuchtungsstrahlengang, wie in Form eines motorbetriebenen, radförmigen Projektionsgitters erzeugt wird. Durch eine fehlerhafte Ansteuerung beziehungsweise einen fehlerhaften Antrieb der mechanisch angetriebenen Projektionsgitter kann es daher zu Positionierungsfehlern kommen, die im Ergebnis zu fehlerhaften dreidimensionalen Bilddaten des Objekts führen.

Ein weiterer Nachteil besteht darin, dass die genannten Verfahren lediglich eine dreidimensionale Vermessung des Objekts und keine Farbmessung ermöglichen.

Die Aufgabe der vorliegenden Erfindung besteht daher darin, eine Kamera bereit zu stellen, die kompakt aufgebaut ist, eine fehlerfreie Vermessung des dentalen Objekts und eine Farbmessung ermöglicht.

### Darstellung der Erfindung

Die Erfindung betrifft eine Kamera zur dreidimensionalen Vermessung eines dentalen Objekts, aufweisend mindestens eine Lichtquelle, die einen Beleuchtungsstrahl abstrahlt, mindestens eine Projektionsmaske, die ein Projektionsmuster erzeugt, eine Fokussierungsoptik, die das Projektionsmuster in einer scharfen Ebene in einem festgelegten Fokusabstand relativ zur dentalen Kamera abbildet, wobei das auf das Objekt projizierte Projektionsmuster als ein Beobachtungsstrahl vom Objekt zurückgestrahlt wird und mittels eines Sensors aufgenommen wird. Bei der Vermessung des Objekts wird die Fokussierungsoptik so gesteuert, dass der Fokusabstand der scharfen Ebene relativ zur Kamera schrittweise zwischen mehreren festgelegten Scanpositionen verstellt wird. Dabei ist im Strahlengang des Beobachtungsstrahls vor dem Sensor eine Beobachtungsmaske angeordnet, wobei die Beobachtungsmaske starr relativ zur Projektionsmaske ausgerichtet ist. Die Projektionsmaske ist aus mehreren Projektionsmusterelementen aufgebaut, die unterschiedliche Farbfilter enthalten. Die Beobachtungsmaske ist ebenfalls aus mehreren Beobachtungsmaskenelementen aufgebaut, die unterschiedliche Farbfilter enthalten.

Die Verstellung der Fokussierungsoptik kann kontinuierlich erfolgen, wobei lediglich die Bilddaten der Aufnahmen diskret an den festgelegten Scanpositionen ausgelesen werden.

Die Kamera kann in ein herkömmliches Gehäuse in Form eines Handstücks integriert sein. Die Lichtquelle kann beispielsweise eine weiße LED oder eine Gruppe von farbigen LED's sein, die einen Beleuchtungsstrahl mit einem breiten Spektrum abstrahlt. Das Projektionsmittel kann ein Gitter aus mehreren Farbfiltern oder auch ein farbiger digitaler Lichtprojektor aus Flüssigkristallelementen (LCD) sein, der entsprechend angesteuert wird und das Projektionsmuster erzeugt. Die Fokussierungsoptik ist verstellbar und fokussiert das Projektionsmuster auf die festgelegte scharfe Ebene, wobei die scharfe Ebene schrittweise verändert wird, sodass das Objekt vollständig abgescannt wird. Die Scanpositionen können beispielsweise einen Abstand von 0,1 mm zueinander aufweisen.

Die vorliegende Kamera funktioniert nach einem Verfahren, das eine Kombination aus einem tiefscannenden konfokalen dreidimensionalen Messverfahren mit einer Farbmatrix darstellt. Die Projektionsmaske kann bezüglich der Anordnung der Farbfilter mit der Beobachtungsmaske übereinstimmen, wobei ein Projektionsmaskenelement beziehungsweise das entsprechende Beleuchtungsmaskenelement einem einzelnen Pixel des Sensors oder beispielsweise einer 2x2-Gruppe aus vier Pixeln oder beispielsweise einer 3x3-Gruppe aus neun Pixeln des Sensors entsprechen kann. Falls das Projektionsmuster also scharf abgebildet wird und die Lage der scharfen Schicht mit der Objektoberfläche des Objekts übereinstimmt wird ein Intensitätswert für ein bestimmtes Pixel maximal, wobei die umgebenden Beobachtungsmaskenelemente bezüglich dieses Pixels eine andere Farbe aufweisen. Falls das Projektionsmuster unscharf abgebildet wird und die Lage der Objektoberfläche nicht mit der Lage der scharfen Schicht übereinstimmt, erscheint das Objekt in den Aufnahmen unscharf. Der Intensitätswert nimmt dann für jedes Beobachtungsmaskenelement ab, wobei diejenigen Beobachtungsstrahlen, die über die Grenzen des Beobachtungsmaskenelements hinaustreten, ausgeblendet werden, da die Farbfilter der umgebenden Beobachtungsmaskenelemente eine andere Farbe aufweisen.

Dadurch entsteht also ein konfokalähnlicher Intensitätsverlauf in einen Pixel, d.h. die Intensität in einen Pixel als Funktion der Fokuslage wird maximal, wenn die Objektlage der Fokuslage entspricht.

Alternativ kann ähnlich einem Depth-from-Fokus-Ansatz der lokale räumliche Kontrast eines Pixels bestimmt werden. Dies kann durch die Auswertung der Intensität eines Pixels relativ zu den Intensitäten der Nachbar Pixel erfolgen. Falls das Objekt in der Fokuslage angeordnet ist, wird also der lokale Kontrast maximal.

Dadurch wird ein lokaler Kontrast einer Signalkurve des Intensitätswertes in Abhängigkeit vom Fokusabstand und damit das Verhältnis zwischen einem Signalmaximum und einem Signaluntergrund verbessert.

Ein weiterer Vorteil dieser Kamera besteht darin, dass durch die Auswertung der Intensitätswerte für die einzelnen Beobachtungsmaskenelemente eine Farbmessung der Objektoberfläche ermöglicht wird.

Vorteilhafterweise können die Abmessungen einer Abbildung eines bestimmten Projektionsmaskenelements des Projektionsmusters in der Ebene der Beobachtungsmaske den Abmessungen eines entsprechenden Beobachtungsmaskenelements entsprechen, wobei ein Farbfilter im Projektionsmaskenelement und ein Farbfilter im entsprechenden Beobachtungsmaskenelement zumindest teilweise einen übereinstimmenden Spektralbereich durchlassen.

Dadurch gelangen lediglich diejenigen Beobachtungsstrahlen des Projektionsmusters zum Sensor, die innerhalb des entsprechenden Beobachtungselements angeordnet sind, wobei diejenigen Beobachtungsstrahlen, die über die Grenzen dieses Beobachtungselements hinaustreten ausgeblendet werden, da die umgebenden Beobachtungsmaskenelemente eine andere Farbe aufweisen und dadurch nur einen abweichenden Spektralbereich durchlassen.

Vorteilhafterweise kann die Lichtquelle eine weiße LED oder eine Kombination aus mehreren farbigen LED's sein, die ein breites Farbspektrum abstrahlt.

Dadurch kann die Lichtquelle einen Beleuchtungsstrahl mit einem breiten Spektrum, wie beispielsweise mit einem tageslichtähnlichen Spektrum, abstrahlen.

Vorteilhafterweise kann die Projektionsmaske und/oder die Beobachtungsmaske aus mehreren optischen Farbfiltern aufgebaut sein oder ein farbiger digitaler Lichtprojektor aus Flüssigkristallenelementen (LCD) sein, der die einzelnen farbigen Projektionsmaskenelemente des Projektionsmusters erzeugt.

Dadurch kann mittels der Projektionsmaske und/oder mittels der Beobachtungsmaske ein beliebig geformtes Projektionsmuster mit der gewünschten Anordnung der farbigen Musterelemente erzeugt werden.

Vorteilhafterweise kann die Projektionsmaske und/oder die Beobachtungsmaske ein schachbrettförmiges Muster aufweisen, wobei die quadratischen Projektionsmaskenelemente und/oder die quadratischen Beobachtungsmaskenelemente lückenlos nebeneinander angeordnet sind.

Dadurch stimmt jedes Projektionsmaskenelement beziehungsweise jedes Beobachtungsmaskenelement in der Abbildungsebene des Sensors mit den Abmessungen eines einzelnen Pixels oder mit den Abmessungen einer quadratischen Pixelgruppe aus vier oder neun Pixeln. Folglich kann für jeden Pixel ein Intensitätswert ausgelesen werden, der einem bestimmten Beobachtungsmaskenelement zugeordnet ist.

Vorteilhafterweise kann die Projektionsmaske und/oder die Beobachtungsmaske aus blauen, grünen, gelben und roten Farbfiltern aufgebaut sein, wobei eine quadratische Vierer-Gruppe einen blauen Farbfilter, einen grünen Farbfilter, einen gelben Farbfilter und einen roten Farbfilter umfasst, sodass jeder Farbfilter keinen benachbarten Farbfilter in der gleichen Farbe aufweist.

Dadurch überschneiden sich die Farbspektren der einzelnen Farbfilter nicht, sodass die vier Farbkanäle separat ausgewertet werden können. Anhand der Intensitätswerte der einzelnen Beobachtungsmaskenelemente in den genannten Grundfarben kann auch eine Farbe des jeweiligen Bereichs der Objektoberfläche bestimmt werden.

Vorteilhafterweise kann die Projektionsmaske und/oder die Beobachtungsmaske so bemessen und ausgerichtet sein, dass jedes Projektionsmaskenelement des Projektionsmusters und/oder jedes entsprechende Beobachtungsmaskenelement auf einen Pixel des Sensors projiziert wird, sodass das projizierte Bild des Musterelements in der Ebene des Sensors den Abmessungen des Pixels entspricht.

Dadurch werden die Auswertung und die Bestimmung der Intensitätswerte vereinfacht. Denn bei diesem Aufbau ist jeder Pixel einem Beobachtungsmaskenelement zugeordnet.

Vorteilhafterweise kann die Projektionsmaske und/oder die Beobachtungsmaske so bemessen und ausgerichtet sein, dass jedes Projektionsmaskenelement des Projektionsmusters und/oder jedes entsprechende Beobachtungsmaskenelement auf eine quadratische Pixelgruppe aus vier Pixeln des Sensors projiziert wird, sodass das projizierte Bild des Projektionsmaskenelements und/oder des Beobachtungsmaskenelements in den Abmessungen dieser Pixelgruppe entspricht.

Dadurch wird zwar die Auflösung im Vergleich zu der vorher genannten Alternative vermindert, jedoch auch die Lichtempfindlichkeit verbessert, sodass die Belichtungszeiten verringert werden können.

Vorteilhafterweise kann bei der Vermessung des Objekts in jeder Scanposition eine Aufnahme erfolgen, wobei unter Verwendung dieser Aufnahme für jedes Projektionsmaskenelement und/oder für jedes entsprechende Beobachtungsmaskenelement ein Intensitätswert ermittelt wird.

Der Intensitätswert wird also unmittelbar durch das Auslesen der einzelnen Pixel ermittelt. Der Sensor kann ein CMOS-Sensor oder ein CCD-Sensor sein.

Vorteilhafterweise kann unter Verwendung des Intensitätswertes in Abhängigkeit vom Fokusabstand für jedes Projektionsmaskenelement und/oder für jedes entsprechende Beobachtungsmaskenelement eine Tiefeninformation einer Objektoberfläche des Objekts mittels einer Recheneinheit ermittelt wird, sodass auf diese Weise dreidimensionale Oberflächendaten des Objekts messbar sind.

Dadurch wird also der Intensitätswert in Abhängigkeit vom Fokusabstand für jedes Beobachtungsmaskenelement ermittelt. Anschließend kann mittels der Recheneinheit ein Fokusabstand im Maximum des Intensitätswerts ermittelt werden, der dem Fokusabstand die Objektoberfläche für das jeweilige Beobachtungsmaskenelement entspricht. Auf diese Weise können die vollständigen dreidimensionalen Oberflächendaten erzeugt werden.

Vorteilhafterweise kann unter Verwendung der Intensitätswerte von mindestens vier benachbarten Projektionsmaskenelementen ein Farbwert mittels der Recheneinheit ermittelt werden, sodass eine Farbmessung des dentalen Objekts ermittelt wird.

Dadurch wird unter Verwendung der einzelnen Intensitätswerte unterschiedlicher Farbfilter die Farbmessung des Objekts ermöglicht. Nach diesem Verfahren erfolgt also die dreidimensionale Vermessung mittels der oben genannten Kamera.

Ein Vorteil dieses Verfahrens liegt darin, dass die dreidimensionale Vermessung mittels der zueinander ausgerichteten Projektionsmaske und der Beobachtungsmaske erfolgt, wobei keine mechanisch beweglichen Bauteile erforderlich sind.

Ein weiterer Vorteil besteht darin, dass zusätzlich zur dreidimensionalen Vermessung auch eine Farbmessung des Objekts ermöglicht wird.

Die Erfindung betrifft weiterhin ein Verfahren zur dreidimensionalen Vermessung eines dentalen Objekts mittels einer Kamera aufweisend mindestens eine Lichtquelle, die einen Beleuchtungsstrahl abstrahlt, mindestens eine Projektionsmaske, die ein Projektionsmuster erzeugt, eine Fokussierungsoptik, die das Projektionsmuster in einer scharfen Ebene in einem festgelegten Fokusabstand relativ zur dentalen Kamera abbildet, wobei das auf das Objekt projizierte Projektionsmuster als ein Beobachtungsstrahl vom Objekt zurückgestrahlt wird und mittels eines Sensors aufgenommen wird. Bei der Vermessung des Objekts wird die Fokussierungsoptik so gesteuert, dass der Fokusabstand der scharfen Ebene relativ zur Kamera schrittweise zwischen mehreren festgelegten Scanpositionen verstellt wird. Im Strahlengang des Beobachtungsstrahls ist dabei vor dem Sensor eine Beobachtungsmaske angeordnet, wobei die Beobachtungsmaske starr relativ zur Projektionsmaske ausgerichtet ist. Die Projektionsmaske ist aus mehreren Projektionsmusterelementen aufgebaut, die unterschiedliche Farbfilter enthalten. Die Beobachtungsmaske ist ebenfalls aus mehreren Beobachtungsmaskenelementen aufgebaut, die unterschiedliche Farbfilter enthalten. Das vom Objekt zurückgestrahlte Projektionsmuster durchstrahlt also die Farbfilter der Beobachtungsmaske bevor dieses mittels des Sensors aufgenommen wird.

Dieses Verfahren ermöglicht also die Vermessung des dentalen Objekts mittels der oben beschrieben Kamera.

Ein Vorteil dieses Verfahrens besteht also darin, dass die dreidimensionale Vermessung des Objekts ohne bewegliche Teile ermöglicht wird. Dadurch wird also die Fehleranfälligkeit des Systems verbessert.

Ein weiterer Vorteil dieses Verfahrens besteht darin, dass zusätzlich zur dreidimensionalen Vermessung eine Farbmessung des Objekts ermöglicht wird.

Vorteilhafterweise können die Abmessungen einer Abbildung eines bestimmten Projektionsmaskenelements des Projektionsmusters in der Ebene der Beobachtungsmaske den Abmessungen eines entsprechenden Beobachtungsmaskenelements entsprechen. Die Farbfilter im Projektionsmaskenelement und im entsprechenden Beobachtungsmaskenelement können dabei zumindest teilweise einen übereinstimmenden Spektralbereich durchlassen.

Dadurch werden die Beobachtungsstrahlen innerhalb eines Beobachtungsmaskenelements zum jeweiligen Pixel des Sensors durchgelassen, wobei die Beobachtungsstrahlen für dieses Beobachtungsmaskenelement, die über die Grenzen des Beobachtungsmaskenelements durch unscharfe Abbildung hinaustreten, ausgeblendet werden, da die umgebenden Farbfilter eine andere Farbe aufweisen.

Vorteilhafterweise kann die Lichtquelle eine weiße LED oder eine Kombination aus mehreren farbigen LED's sein, die ein breites Farbspektrum abstrahlt.

Dadurch kann die Lichtquelle ein breites Spektrum, die ein tageslichtähnliches Spektrum, abstrahlt.

Vorteilhafterweise kann die Projektionsmaske und/oder die Beobachtungsmaske ein schachbrettförmiges Muster aufweisen, wobei die quadratischen Projektionsmaskenelemente und/oder die quadratischen Beobachtungsmaskenelemente lückenlos nebeneinander angeordnet sind.

Dadurch stimmt die gitterförmige Anordnung der Projektionsmaske und/oder der Beobachtungsmaske mit der Anordnung der Pixel auf dem Sensor überein.

Vorteilhafterweise kann die Projektionsmaske und/oder die Beobachtungsmaske aus blauen, grünen, gelben und roten Farbfiltern aufgebaut sein, wobei eine quadratische Vierer-Gruppe einen blauen Farbfilter, einen grünen Farbfilter, einen gelben Farbfilter und einen roten Farbfilter umfasst, sodass jeder Farbfilter keinen benachbarten Farbfilter in der gleichen Farbe aufweist.

Dadurch werden unabhängig voneinander die farbigen Kanäle mit den Grundfarben ausgewertet, wobei jeder Farbfilter keinen benachbarten Farbfilter in der gleichen Farbe aufweist und dadurch die Beobachtungsstrahlen außerhalb dieses Farbfilters ausgeblendet werden.

Vorteilhafterweise kann die Projektionsmaske und/oder die Beobachtungsmaske so bemessen und ausgerichtet sein, dass jedes Projektionsmaskenelement des Projektionsmusters und/oder jedes entsprechende Beobachtungsmaskenelement auf einen Pixel des Sensors projiziert wird, sodass das projizierte Bild des Musterelements in der Ebene des Sensors den Abmessungen des Pixels entspricht.

Dadurch entspricht der ausgelesene Intensitätswert eines Pixels einem einzelnen Beobachtungsmaskenelement. Somit werden also die Auswertung und die Bestimmung der Tiefeninformationen des Objekts vereinfacht.

Vorteilhafterweise kann bei der Vermessung des Objekts in jeder Scanposition eine Aufnahme erfolgen, wobei unter Verwendung dieser Aufnahme für jedes Projektionsmaskenelement und/oder für jedes entsprechende Beobachtungsmaskenelement ein Intensitätswert ermittelt wird.

Vorteilhafterweise kann unter Verwendung des Intensitätswertes in Abhängigkeit vom Fokusabstand für jedes Projektionsmaskenelement und/oder für jedes entsprechende Beobachtungsmaskenelement eine Tiefeninformation einer Objektoberfläche des Objekts mittels einer Recheneinheit ermittelt werden, sodass auf diese Weise dreidimensionale Oberflächendaten des Objekts vermessen werden.

Dabei wird also der Fokusabstand im Maximum des Intensitätswertes bestimmt, wobei dieser Fokusabstand der Tiefeninformation der Objektoberfläche im Bereich dieses Beobachtungsmaskenelements entspricht.

Vorteilhafterweise kann unter Verwendung der Intensitätswerte von mindestens vier benachbarten Projektionsmaskenelementen ein Farbwert mittels der Recheneinheit ermittelt werden, sodass eine Farbmessung des dentalen Objekts ermittelt wird.

Dadurch wird also auf eine einfache Art und Weise zusätzlich zur dreidimensionalen Vermessung eine Farbmessung ermöglicht. Anhand der Verhältnisse der Grundfarben der benachbarten Farbfilter kann also ein Farbwert berechnet werden, der dem Farbwert im Bereich der jeweiligen Objektoberfläche entspricht.

### Kurzbeschreibung der Zeichnungen

Die Erfindung wird anhand der Zeichnungen erläutert. Es zeigt, die
- Fig. 1: eine Skizze einer Kamera zur dreidimensionalen Vermessung eines dentalen Objekts, die
- Fig. 2: eine Skizze der Projektionsmaske mit mehreren Farbfiltern; die
- Fig. 3: eine Skizze zur Verdeutlichung des Aufbaus der Beobachtungsmaske aus mehreren Farbfiltern; die
- Fig. 4: eine Skizze einer seitlichen Darstellung der Projektionsmaske; die
- Fig. 5: einen Intensitätswert eines bestimmten Beobachtungsmaskenelements; die
- Fig. 6: eine Skizze der Intensitätswerte einer Vierer-Gruppe der Farbfilter der Beobachtungsmaske.

### Ausführungsbeispiele

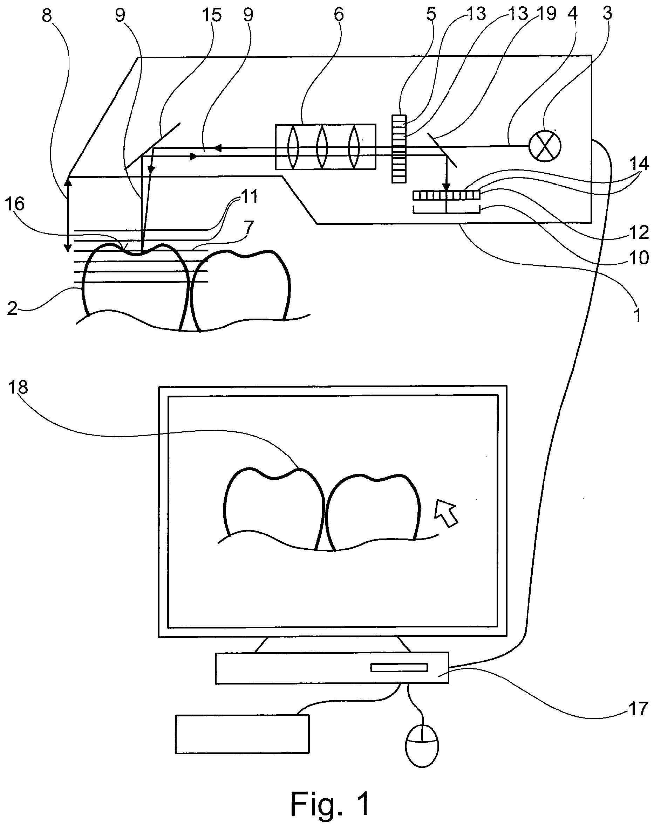

Die Fig. 1 zeigt eine Skizze einer Kamera 1 zur dreidimensionalen Vermessung eines dentalen Objekts 2, wie eines Zahns, wobei die Kamera mindestens eine Lichtquelle 3 aufweist, die ein Beleuchtungsstrahl 4 abstrahlt. Die Lichtquelle 3 kann beispielsweise eine weiße LED oder eine Kombination aus mehreren farbigen LED's sein, die ein breites Farbspektrum abstrahlt.

Die Kamera weist darüber hinaus eine Projektionsmaske 5 auf, die ein Projektionsmuster erzeugt. Eine Fokussierungsoptik 6 bildet die Beleuchtungsstrahlen 4 in einer scharfen Ebene 7 in einem festgelegten Fokusabstand 8 relativ zu der dentalen Kamera 1 scharf ab. Das projizierte Projektionsmuster wird als ein Beobachtungsstrahl 9 vom Objekt 2 zurückgestrahlt, wobei der Beobachtungsstrahl 9 mittels eines Sensors 10, wie eines CCD-Sensors oder eines CMOS-Sensors, aufgenommen wird. Bei der Vermessung des Objekts bzw. während des Scanvorgangs wird die Fokussierungsoptik 6 so gesteuert, dass der Fokusabstand 8 der scharfen Ebene 7 relativ zur Kamera 1 schrittweise zwischen mehreren festgelegten Scanpositionen 11 verstellt wird, die als parallele Linien dargestellt sind. Im Strahlengang des Beobachtungsstrahls 9 ist vor dem Sensor 10 eine Beobachtungsmaske 12 angeordnet, wobei die Beobachtungsmaske 12 relativ zur Projektionsmaske 5 starr ausgerichtet ist. Die Projektionsmaske 5 ist aus mehreren Farbfiltern 13 unterschiedlicher Farbe aufgebaut. Die Beobachtungsmaske ist ebenfalls aus mehreren Farbfiltern 14 unterschiedlicher Farbe aufgebaut. Der Beleuchtungsstrahl 4 wird mittels eines Strahlteilers 15 zum Objekt 2 hin umgelenkt. Die Anordnung der Farbfilter 13 und der Projektionsmaske 5 entspricht der Anordnung der Farbfilter 14 in der Beobachtungsmaske, so dass falls das Projektionsmuster scharf abgebildet wird und die Lage der scharfen Schicht 7 mit einer Oberfläche 16 des Objekts 2 übereinstimmt, wird ein Intensitätswert eines entsprechenden Pixels oder einer Pixelgruppe auf den Sensor 10 maximal, da die umgebenden Farbfilter 14 der Beobachtungsmaske 12 eine andere Farbe aufweisen. Falls das Projektionsmuster unscharf abgebildet wird und die Lage der Oberfläche 16 des Objekts 2 nicht mit der Lage der scharfen Schicht 7 übereinstimmt, erscheint das Objekt in den Aufnahmen unscharf, so dass der Intensitätswert abnimmt.

Denn diejenigen Beobachtungsstrahlen, die über die Grenzen eines Beobachtungsmaskenelements hinaustreten, werden durch die benachbarten Farbfilter 14 einer abweichenden Farbe ausgeblendet. Auf diese Weise wird also der Intensitätswert für jeden Pixel in Abhängigkeit vom Fokusabstand gemessen, so dass für jeden Pixel eine Tiefeninformation der Oberfläche 16 des Objekts ermittelt werden kann. Auf diese Weise wird mittels einer Recheneinheit 17, wie eines Computers, anhand der Bilddaten des Sensors 10 ein dreidimensionales Modell 18 des gesamten Objekts 2 berechnet. Zusätzlich zur dreidimensionalen Vermessung wird durch die Auswertung der Intensitätswerte der einzelnen Farbfilter auch eine Farbmessung der Oberfläche 16 des Objekts 2 ermöglicht.

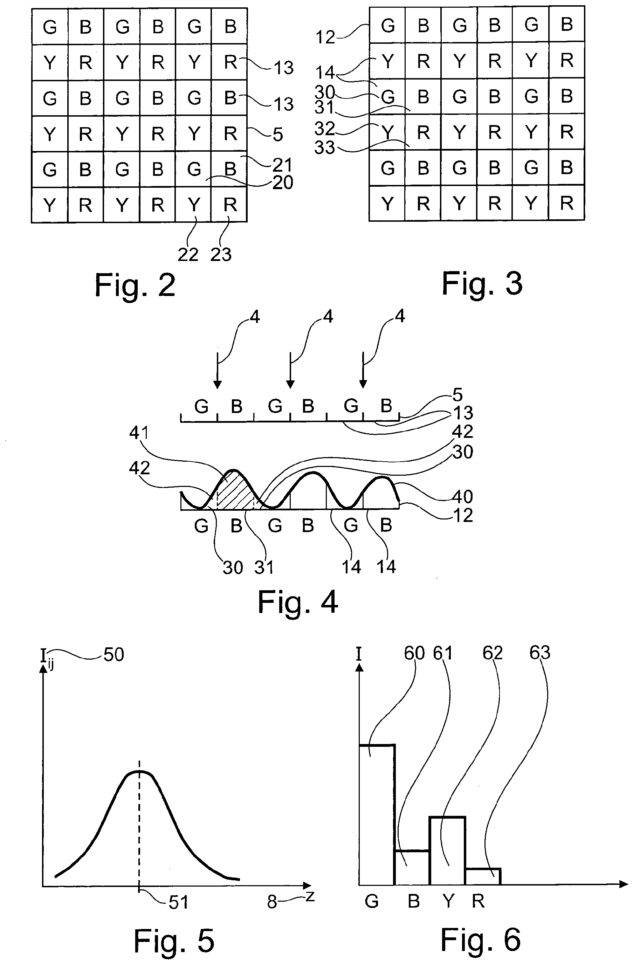

Die Fig. 2 zeigt eine Skizze der Projektionsmaske 5 mit einem schematischen Aufbau aus mehreren Farbfiltern 13. Die Farbfilter sind in quadratischen Vierer-Gruppen bestehend aus einem grünen Farbfilter 20, gekennzeichnet mit G, einem blauen Farbfilter 21, gekennzeichnet mit einem B, einem gelben Farbfilter 22, gekennzeichnet mit einem Y und einem roten Farbfilter 23, gekennzeichnet mit einem R.

Dadurch weist also jeder Farbfilter 13 keinen benachbarten Farbfilter in der gleichen Farbe auf.

Der Beobachtungsstrahl 9 durchstrahlt die Fokussierungsoptik 6 und wird mittels des Strahlteilers 19 zum Sensor 10 umgelenkt.

Die Fig. 3 zeigt eine Skizze zur Verdeutlichung des Aufbaus der Beobachtungsmaske 12 aus Fig. 1 besteht aus mehreren Farbfiltern 14, wobei die Anordnung der grünen Farbfilter 30, der blauen Farbfilter 31, der gelben Farbfilter 32 und der roten Farbfilter 33 mit der Anordnung der Projektionsmaske 5 aus Fig. 2 übereinstimmt.

Die Fig. 4 zeigt eine Skizze einer seitlichen Darstellung der Projektionsmaske 5 mit den Farbfiltern 13 und der Beobachtungsmaske 12 mit den Farbfiltern 14 zur Verdeutlichung der Funktionsweise des vorliegenden Verfahrens. Die Beleuchtungsstrahlen 4 mit einem breitbandigen Spektrum treffen auf die Projektionsmaske, wobei skizzenhaft eine Intensitätsverteilung 40 des blauen Spektrums dargestellt ist. Im vorliegenden Fall wird das Projektionsmuster unscharf abgebildet, da die scharfe Schicht nicht mit der Oberfläche 16 des Objekts 2 aus Figur 1 übereinstimmt, aber so dass lediglich ein erster Anteil 41, der gestrichelt dargestellt ist, am blauen Filter 31 der Beobachtungsmaske 12 ankommt. Ein zweiter Anteil 42 der Intensität des blauen Lichts wird von den benachbarten grünen Farbfiltern 30 ausgeblendet, so dass bei einer scharfen Abbildung des Projektionsmusters die Intensität des blauen Kanals maximal wird und bei einer unscharfen Abbildung abnimmt. Auf diese Weise wird für jeden Kanal, nämlich für die grünen, blauen, gelben und roten Farbfilter die Intensitätswerte in Abhängigkeit vom Fokusabstand 8 ermittelt.

Die Fig. 5 zeigt einen Intensitätswert 50 eines bestimmten Beobachtungsmaskenelements, wie eines grünen, blauen, gelben oder roten Farbfilters der Beobachtungsmaske 12 in Abhängigkeit vom Fokusabstand 8, wobei der Verlauf des Intensitätswerts ein Maximum 51 aufweist, das in der scharfen Schicht liegt und damit eine Tiefeninformation für den jeweiligen Messpunkt auf der Oberfläche 16 des Objekts 2 darstellt. Auf diese Weise werden die einzelnen Tiefeninformationen für alle Messpunkte des Objekts 2 ermittelt und das dreidimensionale Modell 18 aus Fig. 1 berechnet.

Die Fig. 6 zeigt eine Skizze der Intensitätswerte einer Vierer-Gruppe der Farbfilter 30, 31, 32 und 33 der Beobachtungsmaske 12 aus Fig. 3. Aus einem ersten Intensitätswert 60 des grünen Farbfilters 30, einem zweiten Intensitätswert 61 des blauen Farbfilters 31, einem dritten Intensitätswert 62 des gelben Farbfilters 32 und einem vierten Intensitätswert 63 des roten Farbfilters 33 kann dann die Farbe der Oberfläche 16 des Objekts 2 an einem bestimmten Messpunkt ermittelt werden. Auf diese Weise kann also eine vollständige Farbvermessung des Objekts 2 erfolgen.

### Bezugszeichen

- 1: Kamera
- 2: Objekt
- 3: Lichtquelle
- 4: Beleuchtungsstrahl
- 5: Projektionsmaske
- 6: Fokussierungsoptik
- 7: scharfe Ebene
- 8: Fokusabstand
- 9: Beobachtungsstrahl
- 10: Sensor
- 11: Spanposition
- 12: Beobachtungsmaske
- 13: Farbfilter
- 14: Farbfilter
- 15: Strahlteiler
- 16: Oberfläche
- 17: Recheneinheit
- 18: Strahlteiler
- 20: grüner Farbfilter
- 21: blauer Farbfilter
- 22: gelber Farbfilter
- 23: roter Farbfilter
- 30: grüner Farbfilter
- 31: blauer Farbfilter
- 32: gelber Farbfilter
- 33: roter Farbfilter
- 40: Intensitätsverteilung
- 41: erster Anteil
- 42: zweiter Anteil
- 50: Intensitätswert
- 51: Maximum des Intensitätswerts
- 60: erster Intensitätswert
- 61: zweiter Intensitätswert
- 62: dritter Intensitätswert
- 63: vierter Intensitätswert

## Patentansprüche

1. Kamera (1) zur dreidimensionalen Vermessung eines dentalen Objekts (2), aufweisend mindestens eine Lichtquelle (3), die einen Beleuchtungsstrahl (4) abstrahlt, mindestens eine Projektionsmaske (5), die ein Projektionsmuster erzeugt, eine Fokusierungsoptik (6), die das Projektionsmuster in einer scharfen Ebene (7) in einem festgelegten Fokusabstand (8) relativ zur dentalen Kamera (1) abbildet, wobei das auf das Objekt (2) projizierte Projektionsmuster als ein Beobachtungsstrahl (9) vom Objekt (2) zurückgestrahlt wird und mittels eines Sensors (10) aufgenommen wird, wobei die Fokussierungsoptik (6) steuerbar ist, sodass bei der Vermessung des Objekts (2) der Fokusabstand (8) der scharfen Ebene (7) relativ zur Kamera (1) schrittweise zwischen mehreren festgelegten Scanpositionen verstellbar ist, wobei im Strahlengang des Beobachtungsstrahls (9) vor dem Sensor (10) eine Beobachtungsmaske (12) angeordnet ist, wobei die Beobachtungsmaske (12) starr relativ zur Projektionsmaske (5) ausgerichtet ist, wobei die Beobachtungsmaske (12) aus mehreren Beobachtungsmaskenelementen aufgebaut ist, die unterschiedliche Farbfilter (14, 30, 31, 32, 33) enthalten, **dadurch gekennzeichnet, dass** die Projektionsmaske (5) ebenfalls aus mehreren Projektionsmusterelementen aufgebaut ist, die unterschiedliche Farbfilter (13, 20, 21, 22, 23) enthalten.

2. Kamera (1) nach Anspruch 1, **dadurch gekennzeichnet, dass** die Abmessungen einer Abbildung eines bestimmten Projektionsmaskenelements das Projektionsmusters in der Ebene (7) der Beobachtungsmaske (12) den Abmessungen eines entsprechenden Beobachtungsmaskenelements entspricht, wobei ein Farbfilter (13, 20, 21, 22, 23) im Projektionsmaskenelement und ein Farbfilter (14, 30, 31, 32, 33) im entsprechenden Beobachtungsmaskenelement zumindest teilweise einen übereinstimmenden Spektralbereich durchlassen.

3. Kamera (1) nach Anspruch 1 oder 2, **dadurch gekennzeichnet, dass** die Lichtquelle (3) eine weiße LED oder eine Kombination aus mehreren farbigen LEDs ist, die ein breites Farbspektrum abstrahlt.

4. Kamera (1) nach einem der Ansprüche 1 bis 3, **dadurch gekennzeichnet, dass** die Projektionsmaske (5) und/oder die Beobachtungsmaske (12) aus mehreren optischen Farbfiltern (13, 14, 20, 21, 22, 23, 30, 31, 32, 33) aufgebaut ist oder ein farbiger digitaler Lichtprojektor aus Flüssigkristallenelementen (LCD) ist, der die einzelnen farbigen Projektionsmaskenelemente des Projektionsmusters erzeugt.

5. Kamera (1) nach einem der Ansprüche 1 bis 4, **dadurch gekennzeichnet, dass** die Projektionsmaske (5) und/oder die Beobachtungsmaske (12) ein schachbrettförmiges Muster aufweist, wobei die quadratischen Projektionsmaskenelemente und/oder die quadratischen Beobachtungsmaskenelemente lückenlos nebeneinander angeordnet sind.

6. Kamera (1) nach Anspruch 5, **dadurch gekennzeichnet, dass** die Projektionsmaske (5) und/oder die Beobachtungsmaske (12) aus blauen, grünen, gelben und roten Farbfiltern (13, 14, 20, 21, 22, 23, 30, 31, 32, 33) aufgebaut ist, wobei eine quadratische Vierer-Gruppe einen blauen Farbfilter (21), einen grünen Farbfilter (20), einen gelben Farbfilter (22) und einen roten Farbfilter (23) umfasst, sodass jeder Farbfilter (20, 21, 22, 23, 30, 31, 32, 33) keinen benachbarten Farbfilter in der gleichen Farbe aufweist.

7. Kamera (1) nach einem der Ansprüche 1 bis 6, **dadurch gekennzeichnet, dass** die Projektionsmaske (5) und/oder die Beobachtungsmaske (12) so bemessen und ausgerichtet ist, dass jedes Projektionsmaskenelement des Projektionsmusters und/oder jedes entsprechende Beobachtungsmaskenelement auf einen Pixel des Sensors (10) projiziert wird, sodass das projizierte Bild des Musterelements in der Ebene (7) des Sensors (10) den Abmessungen des Pixels entspricht.

8. Kamera (1) nach einem der Ansprüche 1 bis 6, **dadurch gekennzeichnet, dass** die Projektionsmaske (5) und/oder die Beobachtungsmaske (12) so bemessen und ausgerichtet ist, dass jedes Projektionsmaskenelement des Projektionsmusters und/oder jedes entsprechende Beobachtungsmaskenelement auf eine quadratische Pixelgruppe aus vier Pixeln des Sensors (10) projiziert wird, sodass das projizierte Bild des Projektionsmaskenelements und/oder des Beobachtungsmaskenelements in den Abmessungen dieser Pixelgruppe entspricht.

9. Kamera (1) nach einem der Ansprüche 1 bis 8, **dadurch gekennzeichnet, dass** die Kamera eingerichtet ist, in jeder Scanposition (11) eine Aufnahme aufzunehmen, und unter Verwendung dieser Aufnahme für jedes Projektionsmaskenelement und/oder für jedes entsprechende Beobachtungsmaskenelement einen Intensitätswert (50, 60, 61, 62, 63) zu ermitteln.

10. Kamera (1) nach Anspruch 9, **dadurch gekennzeichnet, dass** die Kamera eingerichtet ist, unter Verwendung des Intensitätswertes (50, 60, 61, 62, 63) in Abhängigkeit vom Fokusabstand (8) für jedes Projektionsmaskenelement und/oder für jedes entsprechende Beobachtungsmaskenelement eine Tiefeninformation einer Objektoberfläche (16) des Objekts (2) mittels einer Recheneinheit (17) zu ermitteln, um damit dreidimensionale Oberflächendaten (18) des Objekts (2) zu messen.

11. Kamera (1) nach einem der Ansprüche 1 bis 10, **dadurch gekennzeichnet, dass** die Kamera eingerichtet ist, unter Verwendung der Intensitätswerte (60, 61, 62, 63) von mindestens vier benachbarten Projektionsmaskenelementen einen Farbwert mittels der Recheneinheit (17) zu ermitteln, um eine Farbmessung des dentalen Objekts (2) zu ermitteln.

12. Verfahren zur dreidimensionalen Vermessung eines dentalen Objekts (2) mittels einer Kamera (1) aufweisend mindestens eine Lichtquelle (3), die einen Beleuchtungsstrahl (4) abstrahlt, mindestens eine Projektionsmaske (5), die ein Projektionsmuster erzeugt, eine Fokusierungsoptik, die das Projektionsmuster in einer scharfen Ebene (7) in einem festgelegten Fokusabstand (8) relativ zur dentalen Kamera (1) abbildet, wobei das auf das Objekt (2) projizierte Projektionsmuster als ein Beobachtungsstrahl (9) vom Objekt (2) zurückgestrahlt wird und mittels eines Sensors (10) aufgenommen wird, wobei bei der Vermessung des Objekts (2) die Fokussierungsoptik (6) so gesteuert wird, dass der Fokusabstand (8) der scharfen Ebene (7) relativ zur Kamera (1) schrittweise zwischen mehreren festgelegten Scanpositionen (11) verstellt wird, wobei im Strahlengang des Beobachtungsstrahls (9) vor dem Sensor (10) eine Beobachtungsmaske (12) angeordnet ist, wobei die Beobachtungsmaske (12) starr relativ zur Projektionsmaske (5) ausgerichtet ist, wobei die Projektionsmaske (5) aus mehreren Projektionsmusterelementen aufgebaut ist, die unterschiedliche Farbfilter (13, 20, 21, 22, 23) enthalten, wobei die Beobachtungsmaske (12) ebenfalls aus mehreren Beobachtungsmaskenelementen aufgebaut ist, die unterschiedliche Farbfilter (14, 30, 31, 32, 33) enthalten, wobei das vom Objekt (2) zurückgestrahlte Projektionsmuster die Farbfilter (14, 30, 31, 32, 33) der Beobachtungsmaske (12) durchstrahlt.

13. Verfahren nach Anspruch 12, **dadurch gekennzeichnet, dass** die Abmessungen einer Abbildung eines bestimmten Projektionsmaskenelements des Projektionsmusters in der Ebene (7) der Beobachtungsmaske (12) -den Abmessungen eines entsprechenden Beobachtungsmaskenelements entsprechen, wobei die Farbfilter (13, 14, 20, 21, 22, 23, 30, 31, 32, 33) im Projektionsmaskenelement und im entsprechenden Beobachtungsmaskenelement zumindest teilweise einen übereinstimmenden Spektralbereich durchlassen.

14. Verfahren nach Anspruch 12 oder 13, **dadurch gekennzeichnet, dass** die Lichtquelle (3) eine weiße LED oder eine Kombination aus mehreren farbigen LEDs ist, die ein breites Farbspektrum abstrahlt.

15. Verfahren nach einem der Ansprüche 12 bis 14, **dadurch gekennzeichnet, dass** die Projektionsmaske (5) und/oder die Beobachtungsmaske (12) ein schachbrettförmiges Muster aufweist, wobei die quadratischen Projektionsmaskenelemente und/oder die quadratischen Beobachtungsmaskenelemente lückenlos nebeneinander angeordnet sind.

## Claims

1. Camera (1) for the three-dimensional measurement of a dental object (2), comprising at least one light source (3) that emits an illumination beam (4), at least one projection mask (5) that produces a projection pattern, focusing optics (6) that display the projection pattern in a plane of sharp focus (7) at a defined focal distance (8) relative to the dental camera (1), wherein the projection pattern projected onto the object (2) is reflected by the object (2) as an observation beam (9) and is acquired by means of a sensor (10), wherein the focusing optics (6) can be controlled in such a way that, during the measurement of the object (2), the focal distance (8) of the plane of sharp focus (7) relative to the camera (1) can be adjusted incrementally between a number of defined scan positions, wherein an observation mask (12) is disposed in the beam path of the observation beam (9) in front of the sensor (10), wherein the observation mask (12) is fixedly aligned relative to the projection mask (5), wherein the observation mask (12) consists of a plurality of observation mask elements containing a variety of color filters (14, 30, 31, 32, 33), **characterized in that** the projection mask (5) likewise consists of a plurality of projection pattern elements containing a variety of color filters (13, 20, 21, 22, 23).

2. Camera (1) according to Claim 1, **characterized in that** the dimensions of an image of a specific projection mask element of the projection pattern in the plane (7) of the observation mask (12) correspond to the dimensions of a corresponding observation mask element, wherein a color filter (13, 20, 21, 22, 23) in the projection mask element and a color filter (14, 30, 31, 32, 33) in the corresponding observation mask element at least partially allow a coinciding spectral range to pass through.

3. Camera (1) according to Claim 1 or 2, **characterized in that** the light source (3) is a white LED, or a combination of a number of colored LEDs, that emits a wide color spectrum.

4. Camera (1) according to any of Claims 1 to 3, **characterized in that** the projection mask (5) and/or the observation mask (12) consists of a plurality of optical color filters (13, 14, 20, 21, 22, 23, 30, 31, 32, 33) or is a colored digital light projector consisting of liquid-crystal elements (LCD), which produces the individual colored projection mask elements of the projection pattern.

5. Camera (1) according to any of Claims 1 to 4, **characterized in that** the projection mask (5) and/or the observation mask (12) comprises a checkerboard-like pattern, wherein the square projection mask elements and/or the square observation mask elements are disposed adjacent to one another without gaps.

6. Camera (1) according to Claim 5, **characterized in that** the projection mask (5) and/or the observation mask (12) consists of blue, green, yellow and red color filters (13, 14, 20, 21, 22, 23, 30, 31, 32, 33), wherein a square group of four comprises a blue color filter (21), a green color filter (20), a yellow color filter (22) and a red color filter (23) so that every color filter (20, 21, 22, 23, 30, 31, 32, 33) does not have an adjacent color filter in the same color.

7. Camera (1) according to any of Claims 1 to 6, **characterized in that** the projection mask (5) and/or the observation mask (12) is dimensioned and aligned in such a way that every projection mask element of the projection pattern and/or every corresponding observation mask element is projected onto one pixel of the sensor (10), so that the projected image of the pattern element in the plane (7) of the sensor (10) corresponds to the dimensions of the pixel.

8. Camera (1) according to any of Claims 1 to 6, **characterized in that** the projection mask (5) and/or the observation mask (12) is dimensioned and aligned in such a way that every projection mask element of the projection pattern and/or every corresponding observation mask element is projected onto one square pixel group consisting of four pixels of the sensor (10), so that the projected image of the projection mask element and/or the observation mask element corresponds to the dimensions of said pixel group.

9. Camera (1) according to any of Claims 1 to 8, **characterized in that** the camera is configured to take an image in every scan position (11) and, using this image, determine an intensity value (50, 60, 61, 62, 63) for every projection mask element and/or for every corresponding observation mask element.

10. Camera (1) according to Claim 9, **characterized in that**, by means of an arithmetic unit (17) and using the intensity value (50, 60, 61, 62, 63) as a function of the focal distance (8), the camera is configured to determine depth information of an object surface (16) of the object (2) for every projection mask element and/or for every corresponding observation mask element in order to measure the three-dimensional surface data (18) of the object (2).

11. Camera (1) according to any of Claims 1 to 10, **characterized in that**, with the aid of the arithmetic unit (17) and using the intensity values (60, 61, 62, 63) of at least four adjacent projection mask elements, the camera is configured to determine a color value in order to determine a color measurement of the dental object (2).

12. Method for the three-dimensional measurement of a dental object (2) by means of a camera (1), comprising at least one light source (3) that emits an illumination beam (4), at least one projection mask (5) that produces a projection pattern, focusing optics that display the projection pattern in a plane of sharp focus (7) at a defined focal distance (8) relative to the dental camera (1), wherein the projection pattern projected onto the object (2) is reflected by the object (2) as an observation beam (9) and is acquired by means of a sensor (10), wherein, during the measurement of the object (2), the focusing optics (6) are controlled in such a way that the focal distance (8) of the plane of sharp focus (7) relative to the camera (1) is adjusted incrementally between a number of defined scan positions (11), wherein an observation mask (12) is disposed in the beam path of the observation beam (9) in front of the sensor (10), wherein the observation mask (12) is fixedly aligned relative to the projection mask (5), wherein the projection mask (5) consists of a plurality of projection pattern elements containing a variety of color filters (13, 20, 21, 22, 23), wherein the observation mask (12) likewise consists of a plurality of observation mask elements containing a variety of color filters (14, 30, 31, 32, 33), wherein the projection pattern reflected by the object (2) passes through the color filters (14, 30, 31, 32, 33) of the observation mask (12).

13. Method according to Claim 12, **characterized in that** the dimensions of an image of a specific projection mask element of the projection pattern in the plane (7) of the observation mask (12) correspond to the dimensions of a corresponding observation mask element, wherein the color filters (13, 14, 20, 21, 22, 23, 30, 31, 32, 33) in the projection mask element and in the corresponding observation mask element at least partially allow a coinciding spectral range to pass through.

14. Method according to Claim 12 or 13, **characterized in that** the light source (3) is a white LED, or a combination of a number of colored LEDs, that emits a wide color spectrum.

15. Method according to any of Claims 12 to 14, **characterized in that** the projection mask (5) and/or the observation mask (12) comprises a checkerboard-like pattern, wherein the square projection mask elements and/or the square observation mask elements are disposed adjacent to one another without gaps.

## Revendications

1. Caméra (1) destinée à la mesure en trois dimensions d'un objet dentaire (2), présentant au moins une source de lumière (3) qui émet un rayonnement d'éclairage (4), au moins un masque de projection (5) qui produit un motif de projection, une optique de focalisation (6) qui représente le motif de projection dans un plan net (7) à une distance de focalisation (8) fixe par rapport à la caméra dentaire (1) ; le motif de projection projeté sur l'objet (2) étant réfléchi par l'objet (2) sous la forme d'un rayonnement d'observation (9) et étant enregistré au moyen d'un capteur (10), l'optique de focalisation (6) pouvant être commandée de manière que, lors de la mesure de l'objet (2), la distance de focalisation (8) du plan net (7) par rapport à la caméra (1) puisse être réglée par étapes entre plusieurs positions de balayage fixes, un masque d'observation (12) étant disposé devant le capteur (10) dans la trajectoire du rayonnement d'observation (9), le masque d'observation (12) étant orienté de manière rigide par rapport au masque de projection (5), le masque d'observation (12) étant constitué de plusieurs éléments de masque d'observation contenant différents filtres de couleur (14, 30, 31, 32, 33) ; **caractérisée en ce que** le masque de projection (5) est également constitué de plusieurs éléments de motif de projection contenant différents filtres de couleur (13, 20, 21, 22, 23).

2. Caméra (1) selon la revendication 1, **caractérisée en ce que** les dimensions d'une représentation d'un certain élément de masque de projection du motif de projection dans le plan (7) du masque d'observation (12) correspondent aux dimensions d'un élément de masque d'observation correspondant, un filtre de couleur (13, 20, 21, 22, 23) de l'élément de masque de projection et un filtre de couleur (14, 30, 31, 32, 33) de l'élément de masque d'observation correspondant laissant passer au moins partiellement une plage spectrale concordante.

3. Caméra (1) selon la revendication 1 ou 2, **caractérisée en ce que** la source lumineuse (3) est une DEL blanche ou une combinaison de plusieurs DEL colorées, qui émet un spectre coloré large.

4. Caméra (1) selon une des revendications 1 à 3, **caractérisée en ce que** le masque de projection (5) et/ou le masque d'observation (12) sont constitués de plusieurs filtres de couleur optiques (13, 14, 20, 21, 22, 23, 30, 31, 32, 33) ou sont constitués d'un projecteur numérique de lumière colorée composé d'éléments à cristaux liquides (LCD), qui produit les différents éléments de masque de projection colorés du motif de projection.

5. Caméra (1) selon une des revendications 1 à 4, **caractérisée en ce que** le masque de projection (5) et/ou le masque d'observation (12) présentent un motif en forme de damier, et dans laquelle les éléments de masque de projection quadratiques et/ou les éléments de masque d'observation quadratiques sont disposés sans espace les uns à côté des autres.

6. Caméra (1) selon la revendication 5, **caractérisée en ce que** le masque de projection (5) et/ou le masque
d'observation (12) sont constitués de filtres de couleur bleu, vert, jaune et rouge (13, 14, 20, 21, 22, 23, 30, 31, 32, 33), un groupe quadratique de quatre comprenant un filtre de couleur bleu (21), un filtre de couleur vert (20), un filtre de couleur jaune (22) et un filtre de couleur rouge (23) de telle manière que chaque filtre de couleur (20, 21, 22, 23, 30, 31, 32, 33) ne présente pas de filtre de couleur voisin de la même couleur.

7. Caméra (1) selon une des revendications 1 à 6, **caractérisée en ce que** le masque de projection (5) et/ou le masque d'observation (12) sont dimensionnés et orientés de manière que chaque élément de masque de projection du motif de projection et/ou chaque élément de masque d'observation correspondant soient projetés sur un pixel du capteur (10) de telle manière que l'image projetée de l'élément de motif dans le plan (7) du capteur (10) corresponde aux dimensions du pixel.

8. Caméra (1) selon une des revendications 1 à 6, **caractérisée en ce que** le masque de projection (5) et/ou le masque d'observation (12) sont dimensionnés et orientés de manière que chaque élément de masque de projection du motif de projection et/ou chaque élément de masque d'observation correspondant soient projetés sur un groupe de pixels quadratique constitué de quatre pixels du capteur (10) de telle manière que l'image projetée de l'élément de masque de projection et/ou de l'élément de masque d'observation corresponde aux dimensions de ce groupe de pixels.

9. Caméra (1) selon une des revendications 1 à 8, **caractérisée en ce que** la caméra est agencée de manière à enregistrer un enregistrement dans chaque position de balayage (11) et, au moyen de cet enregistrement, pour déterminer une valeur d'intensité (50, 60, 61, 62, 63) pour chaque élément de masque de projection et/ou chaque élément de masque d'observation correspondant.

10. Caméra (1) selon la revendication 9, **caractérisée en ce que** la caméra est agencée de manière à déterminer, pour chaque élément de masque de projection et/ou chaque élément de masque d'observation correspondant, une information de profondeur d'une surface (16) de l'objet (2) au moyen d'une unité de calcul (17) en exploitant la valeur d'intensité (50, 60, 61, 62, 63) en fonction de la distance de focalisation (8) et pour ainsi mesurer des données de surface tridimensionnelles (18) de l'objet (2).

11. Caméra (1) selon une des revendications 1 à 10, **caractérisée en ce que** la caméra est agencée de manière à déterminer une valeur de couleur au moyen de l'unité de calcul (17) en exploitant les valeurs d'intensité (60, 61, 62, 63) d'au moins quatre éléments de masque de projection voisins, afin de déterminer une mesure de couleur de l'objet dentaire (2).

12. Procédé de mesure en trois dimensions d'un objet dentaire (2) au moyen d'une caméra (1) présentant au moins une source lumineuse (3) qui émet un rayonnement d'éclairage (4), au moins un masque de projection (5) qui produit un motif de projection, une optique de focalisation qui représente le motif de projection dans un plan net (7) à une distance de focalisation (8) fixe par rapport à la caméra dentaire (1), le motif de projection projeté sur l'objet (2) étant réfléchi par l'objet (2) sous la forme d'un rayonnement d'observation (9) et étant enregistré au moyen d'un capteur (10), l'optique de focalisation (6) étant commandée de manière que, lors de la mesure de l'objet (2), la distance de focalisation (8) du plan net (7) par rapport à la caméra (1) soit réglée par étapes entre plusieurs positions de balayage (11) fixes, un masque d'observation (12) étant disposé devant le capteur (10) dans la trajectoire du rayonnement d'observation (9), le masque d'observation (12) étant orienté de manière rigide par rapport au masque de projection (5), le masque de projection (5) étant constitué de plusieurs éléments de motif de projection contenant différents filtres de couleur (13, 20, 21, 22, 23), le masque d'observation (12) étant également constitué de plusieurs éléments de masque d'observation contenant différents filtres de couleur (14, 30, 31, 32, 33), le motif de projection réfléchi par l'objet (2) traversant les filtres de couleur (14, 30, 31, 32, 33) du masque d'observation (12).

13. Procédé selon la revendication 12, **caractérisé en ce que** les dimensions d'une représentation d'un certain élément de masque de projection du motif de projection dans le plan (7) du masque d'observation (12) correspondent aux dimensions d'un élément de masque d'observation correspondant, les filtres de couleur (13, 14, 20, 21, 22, 23, 30, 31, 32, 33) de l'élément de masque de projection et de l'élément de masque d'observation correspondant laissant passer au moins partiellement une plage spectrale concordante.

14. Procédé selon la revendication 12 ou 13, **caractérisé en ce que** la source lumineuse (3) est une DEL blanche ou une combinaison de plusieurs DEL colorées, qui émet un spectre coloré large.

15. Procédé selon une des revendications 12 à 14, **caractérisé en ce que** le masque de projection (5) et/ou le masque d'observation (12) présentent un motif en forme de damier, les éléments de masque de projection quadratiques et/ou les éléments de masque d'observation quadratiques étant disposés les uns à côté des autres sans espace.
